# EUROPEAN PATENT APPLICATION

(11) **EP 4 800 704 A1**
(43) Date of publication of application: **02.09.2026**
(21) Application number: 25160807.1
(22) Date of filing: 28.02.2025
(51) Int. Cl.: G16H 40/20, G06Q 10/0633, G16H 70/20

(54) **CUSTOMIZATION OF A CLINICAL PROTOCOL WORKFLOW**

(71) Applicant: Koninklijke Philips N.V., 5656 AE Eindhoven (NL)
(72) Inventor: DEB, Brijesh, Eindhoven (NL)
(74) Representative: Philips Intellectual Property & Standards

(57) **Abstract**

A method for customizing a clinical protocol workflow by generating a decision model based on a clinical rule specific to a subject. The method comprises obtaining clinical protocol data (110) specific to subject wherein the clinical protocol data comprises a clinical rule; generating a decision model (120) based on the obtained clinical protocol data; adjusting a predetermined general workflow model (130) based on the obtained clinical protocol data to generate an adjusted predetermined general workflow model; and executing the adjusted predetermined general workflow model (140) using the generated decision model.

## Description

### FIELD OF THE INVENTION

This invention relates to the field of clinical protocol workflows and more specifically it relates to customization of clinical protocol workflows.

### BACKGROUND OF THE INVENTION

A clinical protocol defines the tasks (i.e., activities), data and rules (i.e., decisions) of a specific area of care (such as diabetes, COPD, coronary artery disease, heart failure, etc.) for a patient. In general, a clinical protocol is a workflow of tasks and decision-making which supports implementation of a care plan for the patient. Clinical protocols are sometimes also referred to as clinical pathways, clinical workflows, care plans and integrated care pathways.

Many clinical applications facilitate the automation of clinical protocols, wherein healthcare professionals (HCP) can assign one or more clinical protocols to a patient. Once an assigned clinical protocol workflow is initiated, tasks can be assigned and rules automatically evaluated, for example, based on a schedule or an event, as defined in the protocol. Automation of clinical protocols has been found to improve efficiency, care quality, and patient experience.

There are several ways to automate clinical protocol workflows in clinical applications. For example, a general purpose programming language, such as Java or Python, can be used to programmatically implement the workflow logic. This approach, however, is not preferable, however, as any change in existing clinical protocol logic, or the addition of a new clinical protocol, would also need changes in the application code. It is also difficult to custom build more advanced workflow requirements (such as branching, sequencing, parallel processing, state management, etc.).

Another example approach is to use Business Process Model and Notation (BPMN) and Decision Model and Notation (DMN) standards to model clinical protocol workflow and decision-making. These models can be run in workflow engines which manage and monitor the state of activities as defined in the workflow. A disadvantage with this approach, however, is that the clinical protocol workflows implemented in this way cannot be customized as the underlying business process models are pre-defined. For example, a health care professional cannot create a customized clinical protocol and assign it to patients/subjects based on their individual needs, nor can they make patient-specific rule changes.

### SUMMARY OF THE INVENTION

The invention is defined by the independent claims. The dependent claims define advantageous embodiments

According to examples in accordance with an aspect of the invention, there is provided a computer-implemented method for customizing a clinical protocol workflow.

The method comprises: obtaining clinical protocol data specific to a subject, wherein the clinical protocol data comprises a clinical rule; generating a decision model based on the obtained clinical protocol data; adjusting a predetermined general workflow model based on the obtained clinical protocol data to generate an adjusted predetermined general workflow model; and executing the adjusted predetermined general workflow model using the generated decision model.

According to the invention, it is proposed that by obtaining (e.g., generating or receiving (for example, from a health care professional or algorithm)) at least one clinical rule which is specific to a subject (e.g., has been specifically created for and/or assigned to the subject), a decision model can be generated which is based on said at least one clinical rule. Thus, the decision model is personalized for the subject and the generation of a personalized decision model is thereby facilitated which is then used in conjunction with an adjusted predetermined general workflow model to integrate the personalized decision model into a more general automated clinical protocol workflow. The clinical protocol workflow is thus customized based on the at least one clinical rule specific to the subject.

In a simple example, the clinical protocol data may comprise a clinical rule stating that the subject's heart rate should stay below 100bpm but above 80bpm - this clinical rule has been specifically created for and/or assigned to the subject. A decision model can then be generated based on this clinical rule such that using the decision model allows this rule to be automatically evaluated for the subject, either, for example, as part of executing/running a general workflow model or by executing the decision model by itself.

Further, by executing (i.e., running) a predetermined general workflow model using the generated decision model, an efficient and effective way of utilizing the generated decision model is provided such that it can be utilized in combination with a predetermined (i.e., not personalized) general workflow model which can handle aspects of the subject's care which do not need specific personalized attention. Furthermore, by adjusting the predetermined general workflow model based on the clinical protocol data before executing it, the general workflow model can also be customized or personalized to an extent.

Ultimately, an improved method for customizing a clinical protocol workflow is provided. Further, such a method may be implemented at run-time, thus enabling fast, efficient and/or effective workflow adaptation to subject-specific needs. Implementing a method at run-time means executing it while the program/model is actively running, processing data and making decisions dynamically rather than relying on pre-computed results. In other words, the method processes and analyzes data in real-time as it is received, rather than in advance or after collection.

In some embodiments, the clinical protocol data may further comprise at least one of: metadata; and a clinical task to be performed. These are types of patient-specific data which may be of particular use in adjusting the predetermined general workflow model.

In some embodiments, adjusting the predetermined general workflow model may comprise defining one or more parameter values of the predetermined general workflow model. This may be a particularly effective way of adjusting the predetermined general workflow model to personalize it to the subject, for instance, at run-time.

In some embodiments, executing the predetermined general workflow model may comprise referencing the generated decision model when a rule evaluation is required. This may be a particularly efficient and effective way of essentially integrating the generated decision model into a predetermined general workflow model.

In some embodiments, the predetermined general workflow model may comprise at least one business process model and notation, BPMN. A BPMN is an internationally recognized standard general workflow model and therefore can be efficiently implemented and used in a wide variety of situations.

In some embodiments, the business process model and notation may comprise a main model, a task model, and a rule model. The use of all these sub-models may allow for a widely functional and particularly useful BPMN.

In some embodiments, the decision model may comprise a decision model and notation, DMN. A DMN is an internationally recognized standard decision model and therefore can be efficiently implemented and used in a wide variety of situations.

In some embodiments, the clinical rule may comprise at least one of: a comparison rule; a trend comparison rule; a baseline rule; a deviation rule; and a rate of change rule. These are all types of clinical rules which may be of particular use in the monitoring of a subject, and which may be effectively personalized for a subject.

In some embodiments, the clinical rule may be for at least one physiological parameter. Physiological parameters may be particularly useful parameters of the subject to implement personalized rules for, facilitating more effective monitoring of said subject.

According to another aspect of the invention, there is provided a computer program comprising code means for implementing the method of any herein disclosed method when said program is run on a processing system.

According to another aspect of the invention, there is provided a system for customizing a clinical protocol workflow. The system comprising a processor configured to: obtain clinical protocol data specific to a subject, wherein the clinical protocol data comprises a clinical rule; generate a decision model based on the obtained clinical protocol data; adjust a predetermined general workflow model based on the obtained clinical protocol data to generate an adjusted predetermined general workflow model; and execute the adjusted predetermined general workflow model using the generated decision model.

Thus, there may be proposed concepts for customizing a clinical protocol workflow, and this may be done based on an obtained clinical rule specific to the subject.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flow diagram of a method for customizing a clinical protocol workflow according to a proposed embodiment;
Fig. 2 is a simplified block diagram of a system for customizing a clinical protocol workflow according to a proposed embodiment; and
Fig. 3 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to generating a decision model. According to proposed concepts, a number of possible solutions may be implemented separately or jointly. That is, although these possible solutions may be described below separately, two or more of these possible solutions may be implemented in one combination or another.

Embodiments of the invention aim to provide a method for customizing a clinical protocol workflow based on at least one clinical rule specific to the subject.

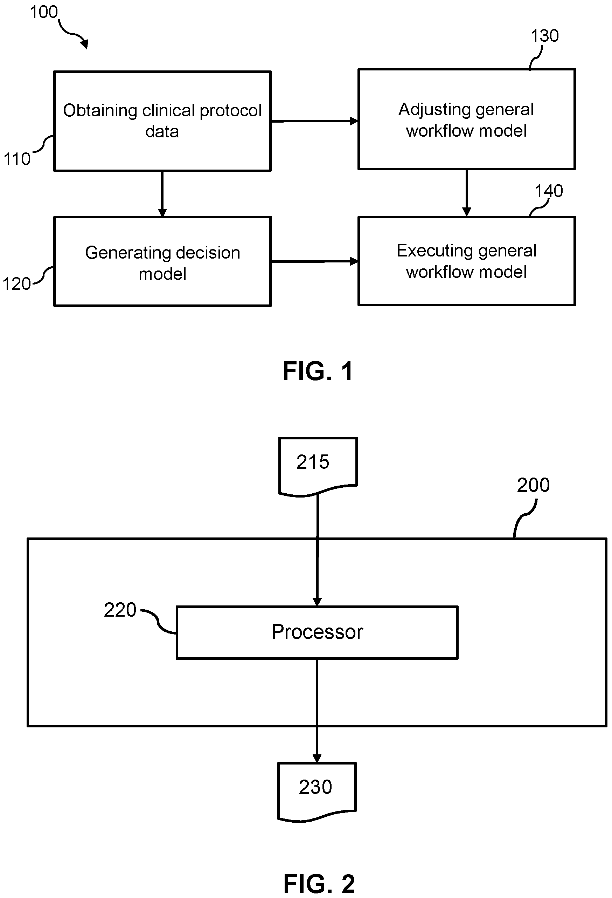

Referring now to Fig. 1, there is depicted a simplified flow diagram of a computer-implemented method 100 for customizing a clinical protocol workflow according to a proposed embodiment.

The method 100 begins with step 110 comprising obtaining (e.g., receiving) clinical protocol data specific to a subject. The clinical protocol data comprises (at least) a clinical rule. A clinical rule is a predefined protocol or decision-making guideline used to standardize medical decision processes, diagnostic criteria, treatment approaches, and/or patient management strategies. A clinical rule can therefore also be referred to as a clinical condition trigger in the sense that if particular data representing the condition of the subject follows or deviates from the rule, follow on actions/procedures can be triggered. For example, if a particular clinical parameter (e.g., sensor/measurement data, a survey score, etc.) goes above/below a predetermined threshold, then a particular response (e.g., an alert, a task to be performed, an additional measurement, etc.) is triggered. A clinical rule can also be referred to as a clinical decision.

**In** an additional example of a clinical rule, if a subject's systolic blood pressure consistently exceeds 140 mmHg and their diastolic pressure is above 90 mmHg across three consecutive measurements, a clinical task to provide antihypertensive medication to the subject can be triggered/assigned.

**In** this embodiment, the clinical rule included in the clinical protocol data comprises at least one of: a comparison rule; a trend comparison rule; a baseline rule; a deviation rule; and a rate of change rule. These are all types of clinical rules which are of particular use in the monitoring of a subject, and which can be effectively personalized for a subject. A comparison rule compares a measurement against a predetermined threshold, and can have actions/consequences associated with whether the measurement is above or below the predetermined threshold. For example, a subject's cholesterol level can be compared to a predetermined threshold of 200 mg/dL - if the subject's cholesterol level is found to be below this, then no action need be taken, however, if the subject's cholesterol level is found to be above this, then an alert can be generated. For example, a trend comparison rule checks sequential measurements to identify directional changes over time - again, actions can be associated with each of the outcomes, for example, whether the trend is up, down, or neutral/stable. For example, a baseline rule uses an initial measurement as a reference/baseline point for subsequent evaluations. For example, if a subject's weight deviates from their reference/baseline by more than a certain percentage, then an alert can be triggered. For example, a deviation rule identifies deviations from a predetermined threshold, for example, if a subject's blood pressure deviates by more than a certain percentage or absolute Figure from a predetermined threshold, then a particular clinical task to be performed can be assigned to a healthcare professional . For example, a rate of change rule monitors the speed and direction of a particular clinical parameter. For example, if a subject's kidney function is found to decline more than 20% in a three-month period, then an alert can be triggered.

Of course, as the skilled person would appreciate, in other embodiments, the clinical rule can comprise any suitable rule for aiding in decision-making regarding a subject's clinical care.

**In** this embodiment, the clinical rule is for at least one physiological parameter. Physiological parameters are particularly useful parameters of the subject to implement personalized rules for, facilitating more effective monitoring of said subject. A physiological parameter represents a measurable characteristic that quantifies a specific bodily function or state of a subject. Example physiological parameters include heart rate, blood pressure, body temperature, respiratory rate, blood glucose level, oxygen saturation, or electrolyte concentrations, etc. As would be understood by the skilled person, however, in other embodiments, the clinical rule need not be for a physiological parameter but rather could be a clinical parameter of another kind, for example, a clinical survey response from the subject or an assessment by a healthcare professional.

It should be noted that the clinical protocol data (and thus the clinical rule) being specific to the subject does not mean that no other subject can share the same rule. It simply means that the rule was specifically assigned to and/or created for the subject, for instance, by a healthcare professional or (machine-learning) algorithm. For example, two separate rules could be specifically created for two separate subjects but coincidentally be the same - this does not mean that the rule is no longer specific to a first subject, however, as it was still specifically created for and assigned to the first subject.

Step 120 comprises generating a decision model based on the obtained clinical protocol data. **In** other words, step 120 comprises programmatically generating a decision model using / in response to the clinical protocol data which comprises at least one clinical rule. **In** yet other words, the at least one clinical rule is converted into a decision model, for example, through programmatic transformation. For instance, the clinical rule may comprise details such as: rule id; trigger type; properties; and (output) flag type; and these details can then be taken and converted into a rule in a (executable) decision model. The skilled person would appreciate the myriad ways in which this could be specifically performed.

It should be noted that that the decision model can also be referred to as specifically a clinical decision model.

**In** this embodiment, the decision model comprises a decision model and notation (DMN). A DMN is an internationally recognized standard decision model and therefore can be efficiently implemented and used in a wide variety of situations. As would be apparent to the skilled person, however, in other embodiments, the decision model can comprise any other form of decision model suitable for automating one or more clinical rules.

**In** a specific example of the invention, the clinical protocol data comprises three comparison rules specifically created for the subject and a decision model is accordingly generated based on these three comparison rules. The generated decision model can then be executed/run and used to process incoming measurements of the subject. For example, when a new measurement is received for the subject (e.g., having patient id: 83430267) with a relevant medical code (e.g., 8867-4, 8480-6, or 29763-7), depending on the type of measurement, the corresponding comparison rule will be evaluated. For example, if measurement data for patient 83430267 is received having medical code 8867-4 and an observation value of 55, the corresponding comparison rule in the decision model stating that the observation value should be below 50 is referenced, and a "high" flag is output accordingly. It should be noted that for another subject, an observation value of 55 may have been perfectly fine, and thus the benefit of being able to implement personalized decision models is made clear.

Step 130 comprises adjusting a predetermined general workflow model based on the obtained clinical protocol data to generate an adjusted predetermined general workflow model. This thus allows the general workflow model to be adjusted based on the obtained subject-specific clinical protocol data such that the general workflow model can also be personalized to an extent. For example, a 'start time' parameter (indicating the date and time at which the workflow model should be executed/started) can be adjusted based on metadata included in the clinical protocol data (e.g., a time and date chosen by a healthcare professional). **In** another example, a 'duration' parameter (indicating the duration for which the workflow should keep running for the subject in hours/days/months) can be adjusted based on one or more aspects of the clinical protocol data which, for example, indicate a health condition of the subject (and thus can inform how long the workflow should run for).

Step 140 comprises executing/running the adjusted predetermined general workflow model using the generated decision model. For example, an instance/version of the adjusted predetermined general workflow model can be executed (i.e., the adjusted predetermined general workflow model can be run) in conjunction with input clinical data (e.g., sensor/measurement data) corresponding to the subject. This provides an efficient and effective way of utilizing the generated decision model such that it can be utilized in combination with an adjusted predetermined (non-personalized) general workflow model which can handle aspects of the subject's care which do not need specific personalized attention.

A general workflow model can be understood as an executable model facilitating the automation of clinical protocols, for example, the sequencing of tasks, decision/rule points, and interactions, etc. **In** other words, a general workflow model allows for clinical protocols to be transformed into computer-executable sequences which can be systematically followed.

Using the generated decision model during a running/executing instance of the predetermined general workflow model can be understood as dynamically applying the decision rules and logic stored within the generated decision model to guide, validate, and/or modify the general workflow model's progression. **In** other words, it can be understood as using/executing the decision model in combination with and/or alongside the general workflow model.

For example, in this embodiment, the generated decision model is used in the sense that the adjusted predetermined general workflow model references the generated decision model when a rule evaluation is required. For example, a rule evaluation would be required when input clinical data corresponding to the subject is received (e.g., by the general workflow model) which includes data relevant to a rule included in the decision model. For instance, the decision model may include a comparison rule for heart rate (e.g., the heart rate should be below X bpm, and if not, then raise an alarm) - therefore, if heart rate data is received, a rule evaluation is required and the decision model would be referenced (i.e., used) to check if the input heart rate follows or deviates from the rule. For example, based on the outcome, a flag can be set (e.g., indicating pass or fail) by the decision model and this flag can then be used by the general workflow model in its decision making. This is a particularly efficient and effective way of essentially integrating the generated decision model into a predetermined general workflow model. As the skilled person would appreciate, of course, in other embodiments, the decision model can be used/referenced in any suitable way during execution of the general workflow model.

It should be noted that the predetermined general workflow model can either be obtained/received from an external source or the method 100 can include actually generating/modelling the general workflow model.

**In** this embodiment, adjusting the predetermined general workflow model comprises defining the value(s) of one or more parameters of the predetermined general workflow model. This may, for example, comprise adjusting existing parameter values that have been previously set/defined for the predetermined general workflow model (i.e. adjusting parameterized values of the general workflow model). **In** other examples, it may comprise setting or defining parameter values for empty/null placeholders within the general workflow model. Adjusting the general workflow model may thus include defining new parameter values, modifying or changing existing parameter values, and/or adding parameter values to empty parameter placeholders. This is a particularly effective way of adjusting the predetermined general workflow model to personalize it to the subject. As would be understood by the skilled person, however, in other embodiments, the predetermined general workflow model can be adjusted based on the clinical protocol data in any other suitable way.

**In** some embodiments, as in this one, the clinical protocol data can further comprise at least one of: metadata; and a clinical task to be performed. These are types of patient-specific data which can be of particular use in adjusting the predetermined general workflow model. For instance, metadata can comprise (non-measurement) data about the subject, for example, at least one of: subject/patient id; care start date; care end date; and date of birth, etc. A clinical task to be performed can comprise any foreseeable clinical task such as, for example, vital sign measurements, diagnostic test ordering, medication administration, etc. The clinical task to be performed can also include further details such as its priority, a specific healthcare professional it should be assigned to, its start pattern, etc. It is noted that even if the clinical protocol data further includes metadata and/or a clinical task to be performed, the decision model is always generated based on at least the clinical rule(s) in the clinical protocol data.

It should be noted that in this embodiment, the predetermined general workflow model comprises at least one business process model and notation, BPMN. A BPMN is an internationally recognized standard general workflow model and therefore can be efficiently implemented and used in a wide variety of situations. The general workflow model of method 200 may also comprise at least one BPMN. In other embodiments, the general workflow model can comprise any suitable general workflow model and is not limited to a BPMN. Specifically in this embodiment, however, each of the at least one BPMNs comprises a main model, a task model, and a rule model. The use of all these sub-models may allow for a widely functional and particularly useful BPMN.

A main model provides an overarching view of the entire protocol; a task model focuses on specific, atomic units of work within the protocol, detailing individual actions to be performed by human or system actors; and a rule model defines the decision logic, conditions, and rules governing protocol flow, determining how different process paths are selected based on specific criteria or decision points.

In a specific example of the invention, clinical protocols can be customized at run-time based on individual based on individual subject/patient-specific needs using business process models and decision models. For example, a three-stage process is proposed. In stage 1, a main clinical protocol workflow, task workflows, and decision workflows are modelled and parameterized using BPMN. In stage 2, patient specific clinical protocol details are received and the workflow models (clinical protocol, task and decision) are configured based on these details. At least one decision model and notation (DMN) is also generated based on the received rule details (included in the patient specific clinical protocol details). In stage 3, an instance/version of the clinical protocol workflow is created and executed for the subject/patient - rules are thus evaluated using the DMN and tasks executed using the configured workflow models.

In more detail, in stage 1 the lifecycle of a typical (i.e., non-customized/personalized) clinical protocol is modelled using BPMN. This main BPMN model contains task and decision workflows as sub-processes. As an example, a main BPMN for a clinical protocol may contain multiple sub-tasks which are executed in parallel as sub-processes. Further, multiple decisions can be evaluated as sub-processes based on events (e.g., incoming measurement data, survey score, etc.). The clinical protocol may also be terminated based on two events: a timer event which is set for a specific duration, or a stop signal.

Task and decision (i.e., rule) workflows are then modelled using BPMN. These are models which act as reusable templates and can be used across different clinical protocol workflows. For example, a task workflow modelled in BPMN may start immediately or after a certain duration from the start of the clinical protocol. It can cater to repetitive tasks, such as when the same task gets assigned after a regular interval - a repeat cron expression can be used to set the repeat interval.

For example, a decision workflow may include a comparison rule, triggered based on incoming observations which match with defined rules. For example, a comparison rule can compare incoming observations against threshold values and accordingly determine an output (i.e., pass or fail). Based on the rule evaluation, an output flag can be set. A flag is a warning or notification which usually represents something of sufficient significance to warrant a special display. Similarly, DMN models for other clinical rules such as a trend comparison rule, baseline rule, target rule, etc., can be added.

The BPMN models can then be parameterized such that the workflows can be configured at run-time based on patient-specific clinical protocol details. For example, the following model elements can be parameterized to support configuration. For the main BPMN model: "duration" timer event; "execute tasks" sub-process; "evaluate rules" sub-process. For the task BPMN model: "start after" timer event; "repeat after" timer event. For the rule BPMN model: "simple comparison" rule.

In stage 2, all the above-mentioned BPMN workflow models (main clinical protocol, task, and decision/rule) are deployed on a workflow engine. Patient-specific clinical protocol details can then be received, including: protocol metadata such as protocol id, protocol name, patient id, start date, end date, etc.; a list of clinical tasks with details such as task id, task type, name, assigned to, priority, start pattern, etc.; and a list of clinical rules with details such as rule id, trigger type, properties, and flag type, etc.

The rule details in the patient-specific clinical protocol details can be used to programmatically generate a DMN file using a DMN generator. The BPMN model parameters set in stage 1 can also be configured based on the patient-specific clinical protocol details. For example, specific values can be set for "start after" and "repeat after" parameters of the task BPMN models based on the patient-specific clinical protocol details.

The main clinical protocol workflow can then be started/run in a workflow engine along with task and decision workflows as sub-processes. In stage 3, the clinical protocol is executed. For example, execution can start immediately after instance creation or at a scheduled time based on the configuration.

Task workflows are executed as sub-processes based on a schedule or events defined in the clinical protocol. For example, an "assign task" step in the task workflow will assign a specific task and, as such, assigned task details (such as task id, task name, etc.) will be made available such that the recipient (e.g., a healthcare professional) can perform the task.

Decision workflows will also be triggered based on a schedule or events (such as incoming measurement data, survey score, etc.) defined in the clinical protocol. All relevant rules included in the DMN model will be evaluated based on the input data and one or more actions taken as per the rule outputs. For example, for each incoming measurement data of a subject, relevant rules will be evaluated if the medical code is the same as in the DMN input parameter. Based on the output of the DMN, a flag can be set. The main clinical protocol workflow will be terminated at the end of the protocol duration or on receipt of a "stop protocol" signal. This will end the lifecycle of the clinical protocol.

In an even more specific example of the invention, a customized clinical protocol is assigned to a subject which includes one survey task and three simple comparison rules. In this example, Camunda can be used as the workflow engine which supports BPMN and DMN standards. Note however that the following steps of this example would also work with other workflow engines (which support BPMN and DMN standards).

In stage 1, a clinical protocol workflow, a task workflow, and a simple comparison workflow are created using a BPMN modeler (such as a Camunda modeler). Next, all three BPMN models are parameterized - this is so that the parametrized values can be set at run-time based on patient-specific clinical protocol details (input data). Example model parameters are listed below in table I:

**Table I**

| **BPMN model** | **Model Element** | **Parameter** |
|---|---|---|
| Main clinical protocol workflow | "Duration" timer event | Timer Definition Type = Duration |
| | | Timer Definition = ${protocolStartTime} |
| Task workflow | "Start After" timer event | Timer Definition Type = Duration |
| | | Timer Definition = ${taskStartTime} |
| | "Start Immediate" timer event | Timer Definition Type = Duration |
| | | Timer Definition = PT5S |
| | "Repeat=1" sequence | Condition Type = Expression |
| | | Expression = ${endAfter ==1} |
| Simple Comparison rule workflow | "Simple Comparison" activity | Implementation = DMN |
| | | Decision Ref = SimpleComparison-${rule.simpleComparison.protocolId} |

In stage 2, all three BPMN workflow models are then deployed on a workflow engine (such as a Camunda workflow engine). Patient-specific clinical protocol details are then received as JSON input which includes the following details: clinical protocol metadata; survey task details; and simple comparison rule details.

Example clinical protocol metadata is shown in table II:

**Table II**

| | |
|---|---|
| protocolId: | 5654638534 |
| patientId: | 83430267 |
| timeZone: | "America/New_York" |
| name: | "Sample Clinical Protocol" |
| state: | "Active" |
| startDate: | "2023-02-17T00:00:00.470+00:00" |
| endDate: | "2023-03-17T00:00:00.470+00:00" |

Example survey task details are shown in table III:

**Table III**

| |
|---|
| ``` "protocolId": 5654638534, "taskId": 57391755, "taskType": "Survey", "surveyId": "90527c6e-c6f5-4fd9-ad4d-3858a9ccd7a1", "assignedTo": "Patient", "name": "Survey Task ", "priority": "Normal", "start": { "startPattern": "START_OF_PROTOCOL", "previousTaskId": 0, "when": "After", "offset": 1, "offsetUnit": "DAY", "timeOfTheDay": "08:00:00" }, "repeat": { "repeatPattern" : "WEEKLY", "every": 1, "onWeekday": { "days": [ ``` |
| ``` "WEDNESDAY", "FRIDAY", "MONDAY" ] }, "endsAfterPattem": "BY_REPETITION", "endsAfter": 3, "cronExpression": "0 0 12 ? * 2,4,6" } ``` |

Example simple comparison rule details are shown in table IV:

**Table IV**

| |
|---|
| ``` "protocolAssignmentId": 5654638534, "id": 3858859, "ruleTypeId": "R_001", "name": "Pulse Rate", "title": "Rule without tasks", "triggerType": "NEW_MEASUREMENT", "complexCondition": { "type": "node", "not": false "logicOp": "and", "elements": [ { "measurement": { "id": 27177630, "type": "MEASUREMENT", ``` |
| ``` "name": "PulseRate", "partOf": [], "medicalCodes": [ { "id": 217, "system": "http://loinc.org", "description": "Heart rate", "code": "8867-4", "observationId": 27177630, "answerChoices": [] } ], "units": [ { "id": 234, "name": "beats/min", "ucumCode": "/min", "precision": 0, "observationId": 27177630, "likelyRanges": [], "validRange": { "id": 133, "low": 0, "high": 0, "unitId": 234 } ``` |
| ``` ], }, "props": { "conditionType": "simpleComparison", "unitCode": "beats/min", "value": 50, "comparisonOp": "<=" } ``` |

Based on the simple comparison rules included in the JSON (i.e., in table IV), a DMN model is generated, as shown in table V:

**Table V**

| Input | | | | | | | | | Output |
|---|---|---|---|---|---|---|---|---|---|
| patientId | protocolid | ruleId | triggerType | ruleVal ue | rule Valu eAlt | observation Value | unitCo de | medicalCo de | flagPrior ity |
| 8343026 7 | 56546385 34 | 385885 9 | NEW_MEAS UREMENT | 50.0 | 0.0 | <=(ruleValu e) | beats/m in | "8867-4" | High |
| 8343026 7 | 56546385 34 | 385886 0 | NEW _MEAS UREMENT | 120.0 | 0.0 | >=(ruleValu e) | mmHg | "8480-6" | High |
| 8343026 7 | 56546385 34 | 385886 1 | NEW _MEAS UREMENT | 75.0 | 100.0 | [ruleValue..r uleValueAlt ] | kg | "29463-7" | High |

The first row in the DMN table (table V) corresponds to the input data shown in table V. For example, if the incoming heart rate of the patient is more than 50, a "High" flag is set. The other two rows are generated accordingly.

Next, the clinical protocol workflow can be started/run in the workflow engine. For example, the Camunda workflow engine provides API to start the process instance. A list of tasks, rules, and clinical protocol metadata are passed as parameters to this API. These details are thus available as process variables during the lifecycle of the process and are used to configure the workflow at run-time. At the end of stage 2, an instance/version of the clinical protocol is created in the workflow engine.

**In** stage 3, the clinical workflow is started on "startDate" (see table II), i.e., 2023-02-17 in this example.

The "Assign Task" activity of the task workflow can take the form of a Camunda service task implemented as a java program which will send survey task details (such as survey id, survey name, priority, etc.) to a message broker. From the message broker, the survey task details will be delivered through a subject/patient-facing user interface for further action. **In** this example, a survey task (having survey id: "90527c6e-c6f5-4fd9-ad4d-3858a9ccd7a1") is assigned to patient (having patient id: "83430267") one day after protocol start, i.e., on 2023-02-18 at 8 am. The same task assignment is configured to be repeated for 3 weeks on Monday, Wednesday and Friday based on the input cron expression.

The simple comparison rules will be evaluated based on input data in the DMN model for each incoming measurement data event. For example, when a new measurement is received for patient (having patient id: "83430267") with medical code ("8867-4", "8480-6" or "29463-7"), the corresponding rule will get evaluated. So, if measurement data having medical code ("8867-4") and an observation value of 55 beats/min is received for patient ("83430267"), the rule output will be a "High" flag. **In** that case, for example, a "Set Flag" Camunda service task can send flag details to a message broker from where it can be delivered through a clinician facing user interface. Finally, the clinical protocol workflow can be terminated at the end of the protocol duration (2023-03-17, in this example) or when a "stop protocol" signal event is received.

Referring now to Fig. 2, there is depicted a system 200 for generating a decision model according to a proposed embodiment. The system 200 comprises a processor 220.

The processor 220 is configured to perform any herein-disclosed method (e.g., method 100). For example, in this embodiment, the processor 220 is configured to perform method 100, i.e., to: obtain clinical protocol data specific to a subject, wherein the clinical protocol data comprises a clinical rule; generate a decision model based on the obtained clinical protocol data; adjust a predetermined general workflow model based on the obtained clinical protocol data to generate an adjusted predetermined general workflow model; and executing the adjusted predetermined general workflow model using the generated decision model. The input 215 into the system 200 thus comprises the clinical protocol data, and the output 230 of the system 200 comprises the generated decision model.

It should be noted that in some embodiments the system 200 can further comprise an input interface (not shown) in communication with the processor 220 for facilitating the obtaining of the clinical protocol data.

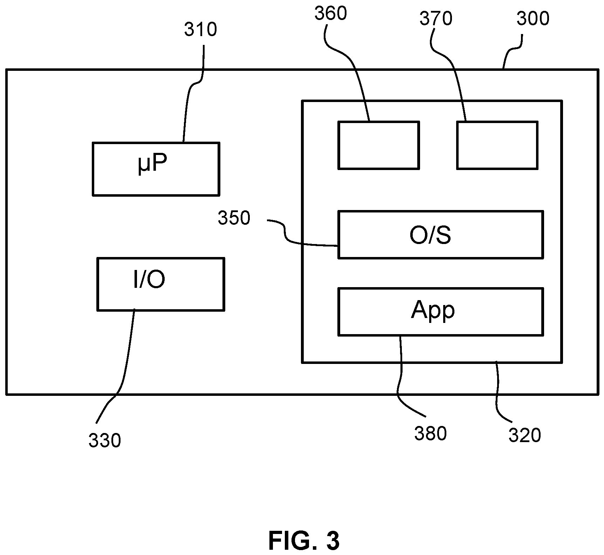

Fig. 3 illustrates an example of a computer 300 within which one or more parts of an embodiment may be employed. Various operations discussed above may utilize the capabilities of the computer 300. In this regard, it is to be understood that system functional blocks can run on a single computer or may be distributed over several computers and locations (e.g. connected via internet).

The computer 300 includes, but is not limited to, PCs, workstations, laptops, PDAs, palm devices, servers, storages, and the like. Generally, in terms of hardware architecture, the computer 300 may include one or more processors 310, memory 320 and one or more I/O devices 330 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 310 is a hardware device for executing software that can be stored in the memory 320. The processor 310 can be virtually any custom made or commercially available processor, a central processing unit (CPU), a digital signal processor (DSP), or an auxiliary processor among several processors associated with the computer 300, and the processor 310 may be a semiconductor based microprocessor (in the form of a microchip) or a microprocessor.

The memory 320 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 320 may incorporate electronic, magnetic, optical, and/or other types of storage media. Note that the memory 320 can have a distributed architecture, where various components are situated remote from one another, but can be accessed by the processor 310.

The software in the memory 320 may include one or more separate programs, each of which comprises an ordered listing of executable instructions for implementing logical functions. The software in the memory 320 includes a suitable operating system (O/S) 350, compiler 360, source code 370, and one or more applications 380 in accordance with exemplary embodiments. As illustrated, the application 380 comprises numerous functional components for implementing the features and operations of the exemplary embodiments. The application 380 of the computer 300 may represent various applications, computational units, logic, functional units, processes, operations, virtual entities, and/or modules in accordance with exemplary embodiments, but the application 380 is not meant to be a limitation.

The operating system 350 controls the execution of other computer programs, and provides scheduling, input-output control, file and data management, memory management, and communication control and related services. It is contemplated by the inventors that the application 380 for implementing exemplary embodiments may be applicable on all commercially available operating systems.

Application 380 may be a source program, executable program (object code), script, or any other entity comprising a set of instructions to be performed. When a source program, then the program is usually translated via a compiler (such as the compiler 360), assembler, interpreter, or the like, which may or may not be included within the memory 320, so as to operate properly in connection with the O/S 350. Furthermore, the application 380 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 330 may include input devices such as, for example but not limited to, a mouse, keyboard, scanner, microphone, camera, etc. Furthermore, the I/O devices 330 may also include output devices, for example but not limited to a printer, display, etc. Finally, the I/O devices 330 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 330 also include components for communicating over various networks, such as the Internet or intranet.

If the computer 300 is a PC, workstation, intelligent device or the like, the software in the memory 320 may further include a basic input output system (BIOS) (omitted for simplicity). The BIOS is a set of essential software routines that initialize and test hardware at start-up, start the O/S 350, and support the transfer of data among the hardware devices. The BIOS is stored in some type of read-only-memory, such as ROM, PROM, EPROM, EEPROM or the like, so that the BIOS can be executed when the computer 300 is activated.

When the computer 300 is in operation, the processor 310 is configured to execute software stored within the memory 320, to communicate data to and from the memory 320, and to generally control operations of the computer 500 pursuant to the software. The application 380 and the O/S 350 are read, in whole or in part, by the processor 310, perhaps buffered within the processor 310, and then executed.

When the application 380 is implemented in software it should be noted that the application 380 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 380 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The method of Fig. 1, and the system of Fig. 2, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 3 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. **In** this regard, each block in the flowchart or block diagrams may represent a module, segment, or portion of instructions, which comprises one or more executable instructions for implementing the specified logical function(s). **In** some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for customizing clinical protocol workflow, the method comprising:
obtaining clinical protocol data (110) specific to a subject, wherein the clinical protocol data comprises a clinical rule;
generating a decision model (120) based on the obtained first clinical protocol data; and
adjusting a predetermined general workflow model (130) based on the obtained clinical protocol data to generate an adjusted predetermined general workflow model; and
executing the adjusted predetermined general workflow model (140) using the generated decision model.

2. The computer-implemented method of claim 1, wherein the clinical protocol data further comprises at least one of: metadata; and a clinical task to be performed.

3. The computer-implemented method of claim 1 or 2, wherein adjusting the predetermined general workflow model comprises defining one or more parameter values of the predetermined general workflow model.

4. The computer-implemented method of any of claims 1 to 3, wherein executing the predetermined general workflow model comprises referencing the generated decision model when a rule evaluation is required.

5. The computer-implemented method of any of claims 1 to 4, wherein the predetermined general workflow model comprises at least one business process model and notation, BPMN.

6. The computer-implemented method of claim 5, wherein the business process model and notation comprises a main model, a task model, and a rule model.

7. The computer-implemented method of any prior claim, wherein the decision model comprises a decision model and notation, DMN.

8. The computer-implemented method of any prior claim, wherein the clinical rule comprises at least one of: a comparison rule; a trend comparison rule; a baseline rule; a target rule; a deviation rule; and a rate of change rule.

9. The computer-implemented method of any prior claim, wherein the clinical rule is for at least one physiological parameter.

10. A computer program comprising code means for implementing the method of any preceding claim when said program is run on a processing system.

11. A system (200) for customizing clinical protocol data generating a decision model, the system comprising:
a processor (220) configured to:
obtain clinical protocol data specific to a subject, wherein the first clinical protocol data comprises a clinical rule;
generate a decision model based on the obtained clinical protocol data;
adjust a predetermined general workflow model based on the obtained clinical protocol data to generate an adjusted predetermined general workflow model; and
execute the adjusted predetermined general workflow model using the generated decision model.

12. The system of claim 11, wherein the clinical protocol data further comprises at least one of: metadata; and a clinical task to be performed.

13. The system of claim 11 or 12, wherein the adjusting of the predetermined general workflow model comprises defining one or more parameter values of the predetermined general workflow model.
